# EUROPÄISCHE PATENTSCHRIFT

(11) **EP 1 251 153 B1**
(45) Veröffentlichungstag und Bekanntmachung des Hinweises auf die Patenterteilung: **13.07.2005**
(21) Anmeldenummer: 02003528.3
(22) Anmeldetag: 15.02.2002
(51) Int. Cl.: C09C 1/62, C09D 5/36, C08K 9/02, C22B 5/20, B41M 3/14

(54) **Weicheisenpigmente**
Soft iron pigments
Pigments de fer doux

(30) Priorität: 23.03.2001 DE 10114445
(43) Veröffentlichungstag der Anmeldung: 23.10.2002
(73) Patentinhaber: ECKART GmbH & Co. KG, 90763 Fürth (DE)
(72) Erfinder: Ostertag, Werner, Dr., 67267 Grünstadt (DE); Trummer, Stefan, Dr., 90480 Nürnberg (DE); Henglein, Frank, Dr., 90409 Nürnberg (DE); Greiwe, Klaus, Dr., 91207 Lauf (DE)
(74) Vertreter: Schneck, Herbert, Dipl.-Phys., Dr.

(56) Entgegenhaltungen:
- EP-A- 0 673 980
- EP-A- 0 949 027
- EP-A- 0 959 108
- WO-A-00/43457
- DE-A- 4 104 310
- DE-A- 4 419 741
- US-A- 4 328 042
- US-A- 5 352 286

## Beschreibung

Die Erfindung richtet sich auf ein Verfahren zur Herstellung von plättchenförmigen, metalloxidbeschichteten Weicheisenpigmenten.

Farbige, metallischen Glanz aufweisende Effektpigmente sind wegen ihrer besonderen optischen Eigenschaften, insbesondere wegen ihrer Brillanz seit Jahren Gegenstand intensiver Forschungs- und Entwicklungsanstrengungen Effektpigmente sind Pigmente von plättchenförmiger Gestalt, die gerichtete Reflexion und nur wenig Streuung aufweisen. Sie können neben Reflexions- auch Interferenzerscheinungen zeigen und müssen anwendungstechnisch in einer Vorzugsrichtung ausgerichtet sein. Die Besonderheit aller mit Effektpigmenten pigmentierten Anwendungen ist die ausgeprägte Winkelabhängigkeit des optischen Eindrucks. Die Partikelgröße von Effektpigmenten übersteigt diejenige von Farbpigmenten beträchtlich. Die bevorzugten, hauptsächlich zur Anwendung kommenden Partikel haben Größen zwischen 5 und 50µm und ein Durchmesser zu Dickenverhältnis von 30 - 150. In einzelnen Anwendungssektoren werden auch Flakes bis zu 250µm im Durchmesser eingesetzt. Vorstellungen zur Idealform metallischer Effektpigmente orientieren sich in der Praxis am so genannten "Silberdollar", einem weitgehend rundlichen, wenig Streuzentren aufweisenden Aluminiumplättchen.

Die vorliegende Erfindung beschreibt ein Verfahren zur Herstellung von Effektpigmenten mit der Schichtenfolge Metalloxid - Eisen - Metalloxid. In der Kategorie metalloxidbeschichteter Eisenpigmente sind bislang nur wenige Entwicklungen bekannt geworden. Diese befassen sich im wesentlichen mit Anlauffarben zeigenden Eisenpigmenten. Als Anlauffarben bezeichnet man Interferenzreflexionsphänomene, die sich bei der Oxidation der Oberfläche von Metallpartikeln ergeben. Bekannte Anlauffarben zeigende Metallpigmente sind die oberflächlich oxidierten Messingpigmente, die je nach Dicke des Oxidbelages im Handel in verschiedenen Farbtönen angeboten werden.

DE 44 19 741 beschreibt Anlauffarben aufweisende Eisenpigmente. Die farbigen metallisch glänzenden Effektpigmente werden durch Verdüsen von schmelzflüssigem Eisen, Mahlen des erhaltenen Eisengrießes und anschließendem Erhitzen der plättchenförmigen Partikel auf 200 - 500°C in Gegenwart von Luftsauerstoff hergestellt. Beim Erhitzen an der Luft bildet sich an der Oberfläche der Eisenpartikel ein Oxidbelag und es zeigen sich Anlauffarben in den Farbtönen Gold, Rotviolett und Blau. Ganz ähnlich wird in EP 673980 die Herstellung farbiger, metallisch glänzender Eisenpigmente beschrieben. Auch hier wird zunächst über Verdüsen von schmelzflüssigem Eisen Eisengrieß hergestellt. Anschließendes Naßmahlen des Eisengrießes und anschließenden Erhitzen der Mahlprodukte bei 350°C ergeben die Anlauffarben Gold, Kupfer, Violett und Blau nacheinander. Für die Farbenfolge ist ein Zeitraum von nur 1 - 4 Minuten notwendig. Die Nachteile von Anlauffarben aufweisenden Eisenpigmenten und dem Verfahren ihrer Herstellung sind vielfältig. Hauptsächlichster Nachteil ist die geringe Reproduzierbarkeit der Farbtöne. Bereits eine geringfügige Veränderung der Dicke des Eisenoxidbelags reicht aus, um andere Interferenzreflexionsfarben hervorzubringen und auch hinsichtlich der Zusammensetzung (Fe₂O₃/Fe₃O₄) ist die durch Oxidation der Eisenoberfläche erzeugte Oxidschicht nicht eindeutig bestimmt. Da reines Eisen in feiner Verteilung pyrophor reagiert, werden die Schwierigkeiten beim Einstellen diskreter Farbtöne mit abnehmender Partikelgröße der Eisenplättchen größer. So wird in EP 673980 auch nur die Herstellung relativ grober Eisenflakes mit Anlauffarben (70 - 80 % der Partikel liegen zwischen 100 - 300µm) aufgezeigt. Weitere Defizite der beschriebenen Verfahren zur Herstellung der o. g. eisenbasierenden Effektpigmente ergeben sich bereits aus der Verdüsungsstufe. Bei der Verdüsung schmelzflüssigen Eisens fallt der Eisengrieß relativ grobteilig und in der Partikelgrößenverteilung wenig homogen an. Da bei Effektpigmenten der Partikelgrößenbereich 5 - 50µm bevorzugt ist, müssen die über Verdüsung hergestellten Eisenpartikel nicht nur verformt, sondern auch zerkleinert werden. Dies ist erheblich aufwendig. Außerdem nimmt bei der Zerkleinerung die Reaktivität der Eisenplättchen erheblich zu.

DE 41 04 310 A1 beschreibt oxidbeschichtete plättchenförmige Pigmente, die nasschemisch durch Beschichten von Edelstahl-Plättchen mit Eisenoxid und Titanoxid hergestellt werden. Die Pigmente zeigen eine stahlgraue bzw. schwarzgraue Körperfarbe und Interferenzfarben. Es werden Stahlplättchen eingesetzt, die in der Regel grobteilig, sehr dick und nicht ferromagnetisch sind.

US-A-5,352,286 beschreibt Eisenplättchen als Pigmentsubstrate, die passiviert und mit Metalloxiden mittels PVD beschichtet werden.

WO 00/43457 beschreibt die Herstellung von Fe2O3-, TiO2- und ZrO2-beschichteten Effektpigmenten, deren metallisches Kernmaterial vorzugsweise aus Titan, Tantal, Zirkon, rostfreiem Stahl oder einer Nickellegierung besteht.

Aus EP-A-0 959 108 ist ein Pigment bekannt, welches jedoch kein Effektpigment ist. Es handelt sich um ein mehrfach beschichtetes, isometrisches Pigment, dessen Kern ein Eisenpulver sein kann. Es wird der Einsatz von Carbonyleisenpulver für weitere Farbbeschichtungen beschrieben. Dieses Carbonyleisenpulver hat jedoch eine isometrische Teilchenform, d. h. es handelt sich um runde Kugeln, und zeigt keinerlei gerichtete Reflexionen, sondern ein Streuverhalten.

In Anbetracht der Schwierigkeiten bei der Herstellung geeigneter Eisensubstrate und deren Oxidationsempfindlichkeit befaßten sich einige Entwicklungen in der Vergangenheit mit der Metalloxidbeschichtung von korrosions- und oxidationsstabilen Edelstahlplättchen. Unter Edelstahl oder "rostfreier Stahl" versteht man Eisenlegierungen mit 18 - 30% Cr, 0 - 8% Ni, daneben Mo, Cu, V und C. Rostfreie Stahlplättchen werden im Handel für Anwendungen in schwerem Korrosionsschutz angeboten. Obgleich Eisen hauptsächlicher Bestandteil der Edelstahlplättchen ist - was des öfteren zur plakativen, aber irreführenden Bezeichnung Eisenplättchen führt - konkurrieren Entwicklungen zur Metalloxid-Beschichtung von Edelstahl- oder rostfreien Stahlplättchen nicht mit dem Gegenstand der vorliegenden Erfindung. Die Gründe sind folgende: Edelstahllegierungen haben andere optische Konstanten als Eisen. Da die optischen Konstanten des Reflektorenmaterials in erheblichem Maße den optischen Gesamteindruck der Pigmente bestimmen, müssen Edelstahl und Eisen unterschieden werden. Weiterhin fehlt Edelstahl die Duktilität des Eisens, weshalb i.a. nur relativ dicke, wenig deckende Plättchen für Beschichtungen zur Verfügung stehen. Edelstahlplättchen werden im Gegensatz zu Eisenplättchen zumeist mechanisch durch spanende Metallbearbeitung hergestellt. Schließlich fehlt Edelstahl der Ferromagnetismus, der Eisen auszeichnet und der die Ursache für die Ausrichtbarkeit von metalloxidbeschichteten Eisenplättchen mit Hilfe eines äußeren Magnetfeldes ist. Der Vollständigkeit halber seien die Anmeldungen, die sich mit der Beschichtung von Edelstahl- oder rostfreien Stahlplättchen befassen, aufgeführt:

DE 41 04 310.3 beschreibt oxidbeschichtete plättchenförmige Pigmente, die naßchemisch durch Beschichten von "stainless steel"-Plättchen mit Eisenoxid und Titanoxid hergestellt werden. Die Pigmente zeigen eine stahlgraue bzw. schwarzgraue Körperfarbe und Interferenzfarben. In ähnlicher Weise wird in JP 10/110 113 die Herstellung titandioxidbeschichteter rostfreier Stahlplättchen beschrieben. WO 00/43 457 beschreibt die Herstellung Fe₂O₃-, TiO₂- und Zr O₂-beschichteter Effektpigmente, deren metallisches Kernmaterial vorzugsweise aus Titan, Tantal, Zirkon, rostfreiem Stahl oder Hastelloy (Nickellegierung) besteht.

Alternative Methoden der Herstellung von metalloxidbelegten Eisenpigmenten über PVD-Methoden und anschließender Zerkleinerung der im Vakuum hergestellten Filme sind vorstellbar. Bislang sind allerdings noch keine nach dieser Methode hergestellten Produkte mit der Schichtenfolge Metalloxid - Eisen - Metalloxid ("Dreischichter" mit Eisenreflektorschicht) bekanntgeworden. Die hohen Kosten dürften einer industriellen Umsetzung dieses Konzeptes entgegenstehen.

Aus dem Dargelegten geht hervor, daß die Entwicklung von Effektpigmenten auf Eisenbasis wesentlich von der Bereitstellung geeigneter Metallsubstrate abhängt. Von den bisher beschriebenen Pigmententwicklungen kann keine die Ansprüche im dekorativen und funktionalen Bereich erfüllen.

Aufgabe der vorliegenden Erfindung ist es, ein Verfahren zur Herstellung neuartiger bunter, metallisch glänzender Effektpigmente für den dekorativen und funktionalen Anwendungsbereich zu entwickeln. Die neuartigen Pigmente sollten sich auszeichnen durch Brillanz und Orientierbarkeit durch ein äußeres Magnetfeld. Sie sollten hohes Deckvermögen aufweisen und Partikelgrößen vorzugsweise im Bereich 5 - 36µm haben. Die neuartigen Pigmente sollten darüber hinaus bei schwach alkalischen Bedingungen, wie sie in vielen Wasserlacken zu finden sind, stabil sein.

Diese Aufgabe wird dadurch gelöst, dass die plättchenförmigen Pigmentsubstrate aus reduziertem Carbonyleisenpulver durch Verformung, insbesondere Mahlung gewonnen werden, und die Oxidbeschichtung aus einem oder mehreren transparenten oder selektiv absorbierenden Metalloxiden besteht. Es wird hochreines reduziertes Carbonyleisenpulver, das in kugeliger Partikelform und enger Partikelgrößenverteilung in diversen mittleren Partikelgrößen von 1 - 10µm kommerziell zur Verfügung steht (Lieferanten: BASF AG, Ludwigshafen oder ISP, Wayne, N. J.), verwendet. Dieses aufgrund seiner Reinheit mechanisch weiche und duktile Pulver wird schonend gemahlen und anschließend der Oxidbeschichtung unterworfen. Die Oxidbeschichtung kann über naßchemische Auffällverfahren oder über CVD-Methoden im Wirbelbett erfolgen. Als vorteilhaft erweist sich, wenn das plättchenförmig verformte Weicheisenpulver oberflächlich passiviert wird. Für die Oxidbeschichtung eignen sich hochbrechende transparente oder teilweise transparente Oxide wie TiO₂, TiO₂₋ₓ, Fe₂O₃, Fe₂O₃₋ₓ oder die Mischphasen von Hämatit mit Al₂O₃, Cr₂O₃ und/oder Mn₂O₃ in besonderer Weise.

Als Ausgangsprodukt für die Herstellung der Substrate für die neuartigen bunten Effektpigmente wurde Weicheisenpulver, wie es bei der Reduktion von Carbonyleisenpulver anfällt, gefunden. Carbonyleisenpulver wird durch Zersetzung von Eisencarbonyldampf hergestellt und ist ein Spezialprodukt der chemischen Industrie. Es fällt in runden Partikeln mit mittleren Partikelgrößen von 1 - 10µm in äußerst enger Partikelgrößenverteilung an und hat zunächst einen Eisengehalt von ca. 96 - 97%. Verunreinigungen sind Kohlenstoff, Sauerstoff und Stickstoff. Die zunächst erhaltenen Partikel sind mechanisch hart. Durch reduzierende Behandlung bei erhöhter Temperatur läßt sich das mechanisch harte Pulver in Weicheisenpulver, das einen Eisengehalt von > 99,0%, besser von > 99,5% zeigt, umwandeln. Die Partikel des hochreinen reduzierten Carbonyleisenpulvers sind weich und leicht mechanisch verformbar (Technisches Merkblatt der BASF, M 5686 d). Sowohl nicht reduziertes als auch reduziertes Carbonyleisenpulver steht in mehreren mittleren Partikelgrößen (1 - 10µm) kommerziell zur Verfügung. Mit reduziertem Carbonyleisenpulver gelingt es, die Eisensubstrate der neuartigen metallisch glänzenden, bunten Effektpigmente, die hohes Deckvermögen, magnetische Permeabilität und gute Stabilität in leicht alkalischen Medien besitzen, herzustellen. Ähnlich feinteiligem hochreinen Aluminiumgrieß, der zur Herstellung von den sogenannten "Silberdollar"-Pigmenten Verwendung findet, ist reduziertes Carbonyleisenpulver mit mittleren Partikelgrößen von 1 - 10µm aufgrund seiner hohen Duktilität besonders gut für die Herstellung von plättchenförmigen Eisensubstraten geeignet. Die plättchenförmige Verformung erfolgt durch Mahlen in Kugelmühlen, wobei kleine Mahlkörper (1 - 5mm) bevorzugt sind. Die Vermahlung kann sowohl durch Naßmahlung unter Verwendung von Testbenzin oder auch durch Trockenvermahlung erfolgen. Zweckmäßigerweise wird zur Vermeidung von Kaltverschweißungen ein Schmiermittel wie zum Beispiel Ölsäure, Stearinsäure oder Phosphoniumverbindungen in kleinen Mengen (0,1 - 3 Gewichtsprozent) zugegeben. Über die Mahldauer und die Auswahl des mittleren Partikeldurchmessers des Ausgangsmaterials lassen sich innerhalb weiter Grenzen Partikeldurchmesser und Formfaktor (Durchmesser zu Dickenverhältnis) der gewünschten Eisensubstrate steuern. Im allgemeinen dauert die Mahlung 1 - 12 Stunden. Nach der Mahlung weisen die metallisch glänzenden Eisenplättchen bereits eine dünne oxidhaltige Passivierungsschicht auf, die sich durch Reaktion der Eisenoberfläche mit Luftsauerstoff oder ubiquitären Wasser bildet. Es hat sich als vorteilhaft erwiesen, die Passivierung der Weicheisenplättchen durch Chromatieren, Phosphatieren, Nitrieren und anderen in der Industrie bekannten Passivierungsmethoden zu verbessern. Die Passivierung führt zur Ausbildung einer sehr dünnen Barriereschicht an der Oberfläche der Weicheisenpartikel. Aufgrund ihrer geringen Dicke (< 20nm) macht diese sich optisch praktisch nicht bemerkbar. Die plättchenförmigen Weicheisenpigmente haben einen dunkleren metallischen Glanz als beispielsweise Aluminiumpigmente. Das Reflexionsvermögen von Eisen liegt im sichtbaren Wellenlängenbereich zwischen 50 und 60 %.

Die Belegung der Oberfläche der plättchenförmigen Weicheisenpartikel mit einer Metalloxidschicht kann aus einem oder mehreren Oxiden der Übergangselemente bestehen. Bevorzugt sind Oxide des Titans, des Eisens und Mischphasen von Oxiden des Eisens mit denen von Chrom und/oder Aluminium und/oder Mangan. Unter den Eisenoxiden besonders bevorzugt ist Hämatit (α-Fe₂O₃). Je höher die Brechzahl des abgeschiedenen Oxids ist, desto geringere Beschichtungsdicken sind notwendig, um Interferenzerscheinungen hervorzurufen.

Wird naßchemisch gearbeitet, so werden zunächst die Hydrolyseprodukte löslicher Metallsalze wie Titanylsulfat, Titantetrachlorid, Eisenchlorid, Chromsulfat usw. auf der Oberfläche der in wässrigem Medium bei erhöhter Temperatur bewegten Weicheisenpartikel abgeschieden. Nach der Abscheidung erfolgt ein Filtrier-, Wasch-, Trocknungs- und Calcinier-Arbeitsgang. Trocknen und Calcinierung müssen schonend, gegebenenfalls im Vakuum oder unter Schutzgas erfolgen, damit es bei der erhöhten Temperatur nicht zur Oxidation der Weicheisensubstrate der Pigmente kommt. Alternativ zur naßchemischen Beschichtung können auch Chemical Vapor Deposition (reaktive CVD-Methoden) zur Herstellung der metalloxidbeschichteten Weicheisenpigmente angewandt werden. Hierbei werden dampfförmige Metallverbindungen wie Eisenpentacarbonyl [Fe(CO)₅] oder TiCl₄ in der Gasphase oxidiert bzw. hydrolisiert und die gebildeten Fe₂O₃-bzw. TiO₂-Aerosole auf den im Gasstrom bewegten Weicheisenplättchen bei erhöhter Temperatur abgeschieden. In der Pigmentindustrie haben sich Wirbelbetten für CVD-Beschichtungen auf Metallplättchen bewährt (US 4,328,042). Über die Steuerung der Dicke der Beschichtung lassen sich gezielt Interferenzfarben herstellen.

Eine zusätzliche Belegung der metalloxidbeschichteten Weicheisenplättchen mit Verbindungen, die die Dispergierbarkeit und das Orientierungsvermögen der Effektpigmente im Medium verbessern, ist möglich. Derartige Belegungen sind koloristisch nicht relevant. Entsprechende Belegungssubstanzen können höhere Fettsäuren sein, aber auch Fettsäure- oder Dicarbonsäurederivate, organische Phosphite und Phosphoniumverbindungen, Phosphorsäureester, Silane, organische und cyklische Amine, Sauerstoff-, Schwefel- oder Stickstoff-enthaltende Heterocyklen, Schwefel-Stickstoff verbindungen höherer Ketone, Alkohole und Aldehyde sowie Gemische derselben.

Aus koloristischer Sicht ist eine Vielzahl von bunten, metallisch glänzenden Pigmentindividuen herstellbar. Die jeweiligen Farbtöne und die Brillanz der Produkte ergeben sich aus den optischen Konstanten der Weicheisensubstrate, den Absorptionskonstanten und der Brechzahl der Metalloxidbeschichtung und der Schichtdicke der Oxidschicht. Für das optische Erscheinungsbild ist die Schichtdicke des Oxidbelags eine wesentlicher Parameter. Er zeigt sich, daß sich die metalloxidbeschichteten Weicheisenpigmente bereits bei relativ geringen Schichtdicke Interferenzerscheinungen einstellen, wie dies bei Interferenzreflexions-Pigmenten typisch ist. Bei Hämatit (α-Fe₂O₃)-beschichteten Weicheisenpigmenten läßt sich Interferenz bereits ab einer Schichtdicke von etwa 20nm beobachten (Gelb). Mit zunehmender Schichtdicke ergeben sich die Interferenzfarben Orange, Rot, Violett, Grün und Blau, dann folgen die Interferenzfarben höherer Ordnung. Voraussetzung für gut wahrnehmbare Interferenzfarben ist hohe Homogenität und Gleichförmigkeit der Beschichtung.

Eine charakteristische Eigenschaft der oxidbeschichteten Weicheisenpigmente ist ihre hohe magnetische Permeabilität. Die Pigmente sind bei der Anwendung daher leicht durch ein äußeres Magnetfeld zu orientieren. Dabei ist es möglich, optisch eindrucksvolle Hell/Dunkel-Muster und Farbtonänderungen zu erzeugen. In der Vergangenheit wurde des öfteren versucht, magnetisch orientierbare Pigmentpartikel über ferrimagnetische Beschichtungen zu erzeugen (Fell-haltiges Fe₂O₃, Fe₃O₄, γ-Fe₂O₃). Dabei zahlte man den Preis, optisch stumpfer und ästhetisch wenig eindrucksvoller Oberflächen. Außerdem war das nutzbare magnetische Moment bei derartigen Pigmenten wesentlich geringer als bei den hochpermeablen Weicheisenpigmentsubstraten, für die es keine Beschränkung in der Beschichtung mit optisch attraktiven Metalloxiden gibt.

Anwendungsgebiet der erfindungsgemäßen Pigmente ist der dekorative Bereich sowie der funktionale Bereich. Hierbei werden die Pigmente im Lack, in Anstrichfarben, Kunststoffen, im Druck, in Glas, Keramik und in der Kosmetik eingesetzt. Im funktionalen Sektor werden die besonderen magnetischen Eigenschaften, die der elektrischen Leitfähigkeit, das Radarwellenabsorptionsvermögen oder das Vermögen der Abschirmung elektromagnetischer Wellen genutzt. Als Beispiel, bei dem es auf die dekorativen und funktionalen Eigenschaften der neuartigen Effektpigmente ankommt, sei der Wertschriftendruck genannt. Hier ermöglicht das Verdrucken der erfindungsgemäßen Pigmente auf Geldscheinen zum einen eine optisch eindrucksvolle, unverwechselbare Markierung der Wertschriften, zum andern vermögen Geldscheinzählmaschinen in Banken, die nach dem Prinzip der Induktion arbeiten, das magnetisch hochpermeable Weicheisensubstrat der Pigmentpartikel zu erfassen.

Die folgenden Versuche erläutern beispielhaft die Erfindung.

### Beispiel 1 A:

### Herstellung von plättchenförmigem Weicheisenpulver

400 g "reduziertes Carbonyleisenpulver" der Firma BASF A.G. Ludwigshafen/Rhein, Deutschland, das die Bezeichnung SQ trägt, wird zusammen mit 0,75 1 Testbenzin und 7 g Stearinsäure in eine Kugelmühle der Dimension 30 cm x 25 cm, die zur Hälfte mit 4 mm großen Stahlkugeln gefüllt ist, gegeben. Das Carbonyleisenpulver SQ hat spezifikationsgemäß einen Eisengehalt von > 99,5% Fe und Partikel in der Größenordnung 4 - 6µm. Die Verunreinigungen werden mit Kohlenstoff > 0,06%, Stickstoff < 0,01% und Sauerstoff < 0,4% angegeben (Technical Leaflet M 5686 e, März 1995). Anschließend wird 4,5 Stunden lang bei 70 Umdrehungen pro Minute gemahlen. Nach Beendigung der Mahlung wird die Mühle entleert, das Mahlpulver von den Mahlkörpern getrennt, abfiltriert, mit Testbenzin gewaschen und danach bei 70° C im Vakuumtrockenschrank getrocknet.

Das erhaltene plättchenförmige Weicheisenpigment zeigt hohen metallischen Glanz und hohe magnetische Permeabilität. Die mittlere Partikelgröße des Produktes wird über Cilas-Messungen (Laserstrahlbeugung) mit 15µm festgestellt. Rasterelektronenmikroskopische Aufnahmen zeigen, daß die Partikel ausgeprägte plättchenförmige Gestalt und ein Durchmesser zu Dikkenverhältnis von ca. 60:1 haben.

### Beispiel 1 B:

### Passivierung der plättchenförmigen Weicheisensubstrate durch CrO₃-Oxidation

300 g der in Beispiel 1 A hergestellten plättchenförmigen Weicheisensubstrate werden in eine Lösung, die aus 600 g Äthylglykol, 400 g Wasser und 30 g CrO₃ besteht, gegeben und bei 70° C eine Stunde lang gerührt.

Danach werden die plättchenförmigen Weicheisensubstrate abfiltriert, mit Ethanol gewaschen und im Vakuumtrockenschrank bei 100° C getrocknet.

### Beispiel 1 C:

### Passivierung der plättchenförmigen Weicheisensubstrate durch SiO₂

300 g der in Beispiel 1 A hergestellten plättchenförmigen Weicheisensubstrate werden in 21 Wasser dispergiert und mit NaOH auf einen pH-Wert von 10 eingestellt. Dann werden 6 g SiO₂ als Natriumsilikat (Wasserglas) zugegeben. Unter Rühren wird durch Zudosieren von 0,1n H₂SO₄-Lösung der pH-Wert über einen Zeitraum von zwei Stunden auf 4 gebracht.

Das SiO₂-passivierte Produkt wird mit Wasser gewaschen und im Vakuumtrockenschrank bei 80° C getrocknet.

### Beispiel 2:

### Oxidbeschichtung von plättchenförmigem Weicheisensubstrat

64,3 g der nach Beispiel 1 C hergestellten, mit SiO₂ passsivierten Weicheisensubstrate werden in einem 250 ml-Drehkolben in 122 g VE-Wasser vorgelegt. Mit HCl wird der PH-Wert auf 3,2 eingestellt. Die Suspension wird auf 75° C aufgeheizt. Nach Erreichen der Temperatur wird unter Rühren eine 28%ige FeCl₃-Lösung mit einer Dosiergeschwindigkeit von 0,11 ml/min über einen Zeitraum von 11 Stunden zudosiert. Dabei wird der pH-Wert durch Zudosieren von 25%iger NaOH konstant gehalten. Die Suspension wird fünf Stunden lang nachgerührt, anschließend filtriert, mit VE-Wasser gewaschen und im Vakuumtrockenschrank bei 95° C über vier Stunden getrocknet.

Das erhaltene Pigment zeigt eine orangegelbe Interferenzfarbe und metallischen Glanz. Eindispergiert in einen Alkyd-Melaminharz-Lack (DIN-Entwurf 53 283) und auf einem Schwarz/Weiß-Karton mit einem Spiralrakel in 100µm Naßfilmdicke abgerakelt, zeigt das Pigment ausgeprägt winkelabhängige Reflexion. Es ist über ein äußeres Magnetfeld orientierbar.

### Beispiel 3:

### Oxidbeschichtung von plättchenförmigem Weicheisensubstrat

500 g des nach Beispiel 1 B hergestellten passivierten Weicheisenpigments werden in 31 Wasser suspendiert und auf 75° C erhitzt. Durch Zugabe von HCl wird der pH-Wert auf 3,3 eingestellt. Dann wird unter Rühren eine 40%ige Fe Cl₃-Lösung mit einer Dosierungsgeschwindigkeit von 90 ml/h zugegeben. Dabei wird der pH-Wert durch Zudosieren von 15%iger NaOH-Lösung bei 3,3 gehalten. Insgesamt werden 450 Eisenchloridlösung zudosiert. Die beschichteten Plättchen werden abfiltriert, mit Wasser gewaschen und im Vakuumtrockenschrank bei 70° C getrocknet. Danach werden sie bei 300° C über 20 min in einem Drehrohr, durch welches Stickstoff geleitet wird, calciniert.

Das erhaltene Pigment hat eine metallische brillantrote Interferenzfarbe und hohes Deckvermögen. Aufgrund seiner magnetischen Eigenschaften ist es durch ein äußeres Magnetfeld leicht zu orientieren. Analysen zeigen, daß die interferenzfähige Eisenoxidschicht rund 40 nm dick ist.

### Beispiel 4:

### Fe₂O₃-Beschichtung im Wirbelbett

In einem Wirbelbettreaktor aus Glas werden 500 g plättchenförmiges Weicheisenpigment, wie es in Beispiel 1, A + B beschrieben ist, eingetragen. Der Wirbelbettreaktor ist mit Infrarotstrahlern beheizbar, hat im unteren Teil einen konischen Wirbelgaseintritt, mechanisch abreinigbare Filterstrümpfe oben und zwei seitliche, auf zwei Drittel Höhe angebrachte Düsen. Es hat eine Innendurchmesser von 6 cm und eine Höhe von 90 cm. Durch die untere Öffnung des Wirbelreaktors wird ein Luft/Stickstoff gemisch im Verhältnis 1:6 zugeführt. Das Gasvolumen wird so lange erhöht, bis sich das Schüttgut aus plättchenförmigem Weicheisenpulver bläht und die Partikel sich schwebend im Bett bewegen. Mit Hilfe der IR-Strahler wird die Temperatur im Innern des Wirbelbettes auf 200° C angehoben. Anschließend wird über die seitlichen Düsen Eisenpentacarbonyldampf, Fe(CO)₅, mit Hilfe eines Trägergases eingetragen. Hierbei wird dergestalt vorgegangen, daß 50 g/h Eisenpentacarbonyl in einem Verdampfer verdampft und mittels 2001 N₂/h (bei 20° C) in den Reaktor transportiert werden. Das Oxidationsprodukt aus der Reaktion von Eisenpentacarbonyl und Luftsauerstoff scheidet sich spontan auf den fluidisierten Weicheisenplättchen ab. Über einen Zeitraum von acht Stunden zeigt das eisenoxidbeschichtete Weicheisenpigment nacheinander die Interferenzfarben gelb, orange, rot, violett, grüngrau, blaugrau, gelb, orange, rot, violett. Nach dem Abbruch der Beschichtung wird das Produkt im Reaktor abgekühlt und diesem entnommen. Über ein gekühltes Rohr lassen sich auch während der Beschichtung kleinere Produktmengen entnehmen.

Die halbstündlich entnommenen Produkte zeigen ohne Ausnahme metallischen Glanz und Interferenzfarben. Die gelben, orangen und roten Interferenzfarben sind von besonderer Brillanz. Aufgrund der inhärenten magnetischen Eigenschaften der Weicheisensubstrate lassen sich die eisenoxidbeschichteten Weicheisenpigmente im Lack leicht ausrichten. Die im Lack applizierten Pigmente weisen ausgeprägte winkelabhängige Reflexion auf. Röntgenaufnahmen zeigen, daß die Beschichtung aus α-Fe₂O₃ besteht. Analysen ergeben, daß die roten Interferenzpigmente 2. Ordnung (Ende der Beschichtungszeit) eine Eisenoxidschichtdicke von etwa 120 nm aufweisen.

## Patentansprüche

1. Verfahren zur Herstellung von plättchenförmigen, metalloxidbeschichteten Weicheisenpigmenten, **dadurch gekennzeichnet, dass** die plättchenförmigen Pigmentsubstrate aus reduziertem Carbonyleisenpulver durch Verformung, insbesondere Mahlung gewonnen werden, und die Oxidbeschichtung aus einem oder mehreren transparenten oder selektiv absorbierenden Metalloxiden besteht.

2. Verfahren nach Anspruch 1, **dadurch gekennzeichnet, dass** die plättchenförmigen Weicheisensubstrate vor der Beschichtung passiviert werden.

3. Verfahren nach Anspruch 1 und 2, **dadurch gekennzeichnet, dass** die Oxidschicht nasschemisch oder durch Chemical Vapor Deposition-Techniken aufgefällt wird.

4. Verfahren nach Annspruch 1 bis 3, **dadurch gekennzeichnet, dass** die Oxidschicht eine Dicke aufweist, die Interferenz-Reflexion ermöglicht.

5. Verfahren nach Anspruch 1 bis 4, **dadurch gekennzeichnet, dass** die Oxidschicht vorzugsweise aus Eisenoxid und/oder eisenoxidbasierenden Mischphasen besteht.

6. Verfahren nach Anspruch 1 bis 5, **dadurch gekennzeichnet, dass** die Pigmente zusätzliche, das Dispergier- und das Orientierungsverhalten verbessernde Beschichtungen aufweisen.

7. Verwendung von oxidbeschichteten Eisenpigmenten, hergestellt nach Anspruch 1 bis 6, im dekorativen und/oder funktionalen Bereich für die Pigmentierung von Lacken, Anstrichen, Kunststoffen, Printmedien, Glas, Keramik und Kosmetik mit magnetische permeablen Effektpigmenten.

8. Verwendung von oxidbeschichteten Eisenpigmenten, hergestellt nach Anspruch 1 bis 6, im Wertschriftendruck.

9. Plättchenförmige, metalloxidbeschichtete Weicheisenpigmente erhalten durch das Verfahren nach einem der Ansprüche 1 bis 6.

## Claims

1. Method for the production of flake shaped, metal oxide coated soft iron pigments, **characterized in that** the flake shaped pigment substrates are obtained from reduced carbonyl iron powder by deformation, particularly by grinding, and the oxide coating consists of one or more transparent or selectively absorbing metal oxides.

2. Method according to claim 1, **characterized in that** the flake shaped soft iron pigments are passivated prior to the coating.

3. Method according to claim 1 and 2, **characterized in that** the oxide layer is deposited either wet-chemically or by means of chemical vapor deposition techniques.

4. Method according to claim 1 through 3, **characterized in that** the oxide layer has a thickness that permits the interference reflection.

5. Method according to claim 1 through 4, **characterized in that** the oxide layer preferably consists of iron oxide and/or iron oxide-based mixed phases.

6. Method according to claim 1 through 5, **characterized in that** the pigments incorporate additional coatings that improve the dispersion and orientation behavior.

7. Use of the oxide coated iron pigments produced according to claim 1 through 6 in the decorative and/or functional field for pigmenting lacquers, paints, plastics, print media, glass, ceramics and cosmetics with magnetically permeable effect pigments

8. Use of the oxide coated iron pigments according to claim 1 through 6 in value printing.

9. Flake shaped, metal oxide coated soft iron pigments obtained by the method according to one of claims 1 through 6.

## Revendications

1. Procédé de préparation de pigments de fer doux lamellaires, revêtus d'oxyde métallique, **caractérisé en ce que** les substrats de pigment lamellaires sont obtenus par déformation, en particulier par broyage, à partir de poudre de fer carbonyle sous forme réduite, et **en ce que** le revêtement d'oxyde consiste en un ou plusieurs oxydes métalliques transparents ou présentant une absorption sélective.

2. Procédé selon la revendication 1, **caractérisé en ce que** les substrats de fer doux lamellaires sont passivés avant d'être revêtus.

3. Procédé selon la revendication 1 et la revendication 2, **caractérisé en ce que** la couche d'oxyde est déposée par un procédé chimique humide ou par une méthode de dépôt chimique en phase vapeur.

4. Procédé selon l'une quelconque des revendications 1 à 3, **caractérisé en ce que** la couche d'oxyde présente une épaisseur qui permet la réflexion par interférence.

5. Procédé selon l'une quelconque des revendications 1 à 4, **caractérisé en ce que** la couche d'oxyde est composée de préférence d'oxyde de fer et/ou de phases mixtes à base d'oxyde de fer.

6. Procédé selon l'une quelconque des revendications 1 à 5, **caractérisé en ce que** les pigments présentent des revêtements supplémentaires qui améliorent le comportement de dispersion et d'oxientation.

7. Utilisation de pigments de fer revêtus d'oxyde, préparés selon les revendications 1 à 6, dans le domaine décoratif et/ou fonctionnel pour la pigmentation des vernis, des peintures, des matières plastiques, de la publicité-presse, du verre, de la céramique et des produits cosmétiques avec des pigments à effet magnétiquement perméables.

8. Utilisation de pigments de fer revêtus d'oxyde, préparés selon l'une quelconque des revendications 1 à 6, dans le domaine de l'impression de sécurité.

9. Pigments de fer doux lamellaires, revêtus d'oxyde métallique, obtenus par le procédé selon l'une quelconque des revendications 1 à 6.
